# EUROPÄISCHE PATENTANMELDUNG

(11) **EP 2 792 296 A1**
(43) Veröffentlichungstag der Anmeldung: **22.10.2014**
(21) Anmeldenummer: 14159027.3
(22) Anmeldetag: 12.03.2014
(51) Int. Cl.: A61B 5/00

(54) **Vorrichtung und Verfahren zur Messung der Kompressibilität eines biologischen Gewebes**

(30) Priorität: 17.04.2013 DE 102013206966
(71) Anmelder: Pohlig GmbH, 83278 Traunstein (DE)
(72) Erfinder: Pohlig, Kurt, 83278 Traunstein (DE); Stahl, Josef, 5091 Unken (AT)
(74) Vertreter: Pfenning, Meinig & Partner GbR

(57) **Zusammenfassung**

Die Erfindung betrifft eine Vorrichtung zur Messung der Kompressibilität eines biologischen Gewebes, insbesondere humanen Gewebes, mit einem Abstützelement zur Abstützung der Vorrichtung auf dem Gewebe, einem gegenüber dem Abstützelement verschiebbar gelagerten Druckelement zur Ausübung eines Drucks auf das Gewebe, und einer Messeinheit zur Bestimmung der Verschiebung des Druckelements relativ zum Abstützelement und hieraus der Kompressibilität des biologischen Gewebes, und ein Verfahren zur Messung der Kompressibilität eines biologischen Gewebes, insbesondere eines humanen Gewebes.

## Beschreibung

Die Erfindung betrifft eine Vorrichtung und ein Verfahren zur Messung der Kompressibilität eines biologischen Gewebes, insbesondere eines humanen Gewebes.

Bei der Herstellung von Orthesen oder Prothesen zur Aufnahme von Gliedmaßen oder äußeren Körperteilen ist es wichtig, den Schaft der Orthese oder der Prothese exakt an das aufzunehmende Körperteil des Patienten anzupassen. Hierzu wird das aufzunehmende Körperteil vermessen und ein entsprechend den Messdaten geformter Schaft hergestellt. Für die exakte Anpassung einer Unterschenkelorthese oder -prothese wird beispielsweise der Umfang des Unterschenkels in verschiedenen Bereichen des Unterschenkels gemessen.

Um einen optimalen Sitz des Schaftes an dem aufzunehmenden Körperteil zu gewährleisten, ist es notwendig die Kompressibilität der Haut zu berücksichtigen. Die Kompressibilität der Haut ist ein Maß dafür, wie weit sich die Haut unter Ausübung eines bestimmten Druckes zusammendrücken lässt. Je nachdem welche Kompressibilität das Gewebe des aufzunehmenden Körperteils aufweist, muss der Schaft der Orthese oder der Prothese beispielsweise enger oder weiter gestaltet sein, um einen optimalen Sitz der Prothese oder der Orthese zu gewährleisten.

Ein Messgerät, mit dem sich reproduzierbare und ausreichend genaue Werte für die Kompressibilität eines biologischen Gewebes erfassen lassen, ist bisher nicht bekannt.

Die Messung der Kompressibilität eines biologischen Gewebes wird beispielsweise mit einer Schieblehre durchgeführt. Hierbei wird ein Teil des Gewebes zwischen den Messschenkeln der Schieblehre erst locker gehalten und dann zusammen gedrückt. Aus der abgelesenen Längendifferenz ergibt sich dann die Kompressibilität des Gewebes.

Nachteilig an diesem Verfahren ist, dass die zur Messung verwendete Hautportion bei jeder Messung variiert und die Schenkel der Schieblehre bei jeder Messung mit unterschiedlicher Kraft das Gewebe zusammendrücken. Dadurch können sich die Messergebnisse für die Kompressibilität bei Wiederholung der Messung stark unterscheiden. Eine reproduzierbare Messung der Kompressibilität ist auf diese Weise nicht möglich.

Im Lichte des Standes der Technik ist es daher die Aufgabe der vorliegenden Erfindung eine Vorrichtung zur Messung der Kompressibilität eines biologischen Gewebes, insbesondere eines humanen Gewebes, bereit zu stellen, mit der reproduzierbare und vergleichbare Messwerte für die Kompressibilität erfasst werden können. Eine weitere Aufgabe der vorliegenden Erfindung ist es ein entsprechendes Verfahren zur Messung der Kompressibilität eines biologischen Gewebes bereitzustellen.

Diese Aufgabe wird durch die Vorrichtung nach Anspruch 1 sowie das Verfahren nach Anspruch 14 gelöst. Vorteilhafte Weiterbildungen der erfindungsgemäßen Vorrichtung werden in den abhängigen Ansprüchen angegeben. Eine erfindungsgemäße Vorrichtung zur Messung der Kompressibilität eines biologischen Gewebes, insbesondere eines humanen Gewebes, weist ein Abstützelement zur Abstützung der Vorrichtung auf dem Gewebe und ein gegenüber dem Abstützelement verschiebbar gelagertes Druckelement zur Ausübung eines Drucks auf das Gewebe auf. Die Vorrichtung weist ferner eine Messeinheit zur Bestimmung der Verschiebung des Druckelements relativ zum Abstützelement auf. Aus dieser Verschiebung kann dann die Kompressibilität des biologischen Gewebes bestimmt werden. Die erfindungsgemäße Vorrichtung ist weiter mit einer Druckerzeugungsvorrichtung versehen. Mittels dieser Druckerzeugungsvorrichtung lässt sich ein einheitlicher Druck auf das Druckelement in Richtung des Gewebes ausüben, wobei dieser Druck im Wesentlichen von der Verschiebung des Druckelementes unabhängig ist.

Die vorliegende Erfindung ermöglicht es, die Kompressibilität eines biologischen Gewebes insbesondere eines humanen Gewebes, verlässlich und reproduzierbar zu ermitteln und vergleichbare Kompressibilitätswerte zu schaffen.

In einer vorteilhaften Ausgestaltung der erfindungsgemäßen Vorrichtung kann die Druckerzeugungsvorrichtung eine Feder enthalten, oder ganz aus einer Feder bestehen. In einer besonders vorteilhaften Ausgestaltung der Erfindung kann die Feder der Druckerzeugungsvorrichtung als Gasdruckfeder ausgebildet sein. Mit einer Gasdruckfeder kann eine konstante Federkennlinie erreicht werden, das heißt die Federkraft ist unabhängig vom Federweg. In diesem Fall wirkt eine Gasdruckfeder beispielsweise mit einem konstanten Druck ihrer Komprimierung entgegen.

Ferner ist es denkbar als Feder der Druckerzeugungsvorrichtung eine Schraubenfeder, eine Öldruckfeder oder eine Elastomerfeder zu verwenden. Es ist auch denkbar, bei der Druckerzeugungsvorrichtung anstatt einer Feder einen Pneumatikzylinder oder einen Gummizug zu verwenden.

Anstatt einer Feder kann die Druckerzeugungsvorrichtung auch eine Kolbenschubeinheit aufweisen oder aus dieser bestehen.

Das Abstützelement kann weiter dahingehend ausgebildet sein, dass es eine flächige Grundplatte zur Auflage auf das biologische Gewebe aufweist. Diese Grundplatte kann insbesondere eine ebene Oberfläche, eine anatomisch geformte Oberfläche oder eine gewölbte Oberfläche aufweisen. In einer weiteren Ausgestaltung der Grundplatte kann die Oberfläche der Grundplatte auch mit einer ringförmigen Gitterstruktur versehen sein. Die Oberfläche der Grundplatte ist also abhängig von der zu messenden Körperregion gestaltet, um möglichst gleichmäßig an dem biologischen Gewebe anzuliegen.

In einer weiteren vorteilhaften Ausgestaltung ist die Vorrichtung derart ausgebildet, dass die Grundplatte einfach austauschbar ist. So können verschieden ausgestaltete Grundplatten je nach zu messender Körperregion mit der Vorrichtung verwendet werden, und es kann eine optimale Auflage der Grundplatte auf der Gewebeoberfläche der jeweiligen Körperregion erreicht werden.

Das Druckelement kann als Druckbolzen oder als Druckstempel ausgebildet sein.

Zur Ausübung des Drucks auf das Gewebe weist das Druckelement bevorzugt eine Kontaktfläche auf. Die Kontaktfläche kann dabei eine Fläche zwischen 0,05 cm² und 10 cm², vorteilhafterweise zwischen 0,05 cm² und 5 cm², besonders bevorzugt zwischen 0,05 cm² und 2 cm², vorteilhafterweise von 0,1 cm² oder 0,5 cm² oder 1 cm² oder 1,5 cm² oder 2 cm² aufweisen.

Die Kontaktfläche kann anatomisch geformt, eben, gewölbt, kreisförmig und/oder oval sein. Die Kontaktfläche kann also an die Form der Oberfläche des zu messenden Gewebes angepasst sein, sodass von der Kontaktfläche ein gleichmäßiger Druck auf das Gewebe ausübbar ist.

Ferner kann die Vorrichtung derart ausgebildet sein, dass das Druckelement einfach austauschbar ist. Dies ist vorteilhaft, da Druckelemente mit verschieden ausgestalteter Kontaktfläche bereitgestellt werden können, die je nach Oberflächenform des zu messenden Gewebes bzw. je nach zu messender Körperregion mit der Vorrichtung verwendet werden können.

Das Abstützelement kann weiter einen Hohlzylinder aufweisen, in dem die Druckerzeugungsvorrichtung derart gelagert, dass die Kraftrichtung der Druckerzeugungsvorrichtung und die Symmetrieachse des Hohlzylinders parallel zueinander liegen.
Alternativ kann die Druckerzeugungsvorrichtung in einem Hohlzylinder, der unabhängig vom Abstützelement sein kann, derart gelagert sein, dass die Kraftrichtung der Druckerzeugungsvorrichtung und die Symmetrieachse des Hohlzylinders parallel zueinander liegen.

Ferner kann innerhalb des Hohlzylinders eine in Richtung der Symmetrieachse bewegliche Kolbenstange oder ein in Richtung der Symmetrieachse beweglicher Kolben angeordnet sein, die bzw. der Teil der Druckerzeugungsvorrichtung ist und mit dem Druckelement derart in Kontakt steht, dass die Druckkraft des Druckerzeugungselementes über die Kolbenstange bzw. den Kolben auf das Druckelement übertragen wird.

In einer bevorzugten Ausgestaltung der Erfindung kann die Messeinheit einen Messstab aufweisen, der zwischen dem Druckelement und dem Hohlzylinder angeordnet ist und mit dem Druckelement und der Druckerzeugungsvorrichtung derart in Kontakt steht, dass er die Druckkraft von der Druckerzeugungsvorrichtung auf das Druckelement überträgt.

An dem Messstab kann noch eine Feststellschraube zur Fixierung der Position des Messstabs in Richtung der Kraftrichtung der Druckkraft angeordnet sein. Der Vorteil hiervon ist, dass das Messergebnis, also die relative Verschiebung des Messstabs, nicht ausschließlich während der Messung ablesbar ist, sondern dass die relative Verschiebung des Messstabs durch die Betätigung der Feststellschraube auch noch nach der Messung aufrecht erhalten werden kann.

Ferner kann die Messeinheit eine Messvorrichtung zur genauen Messung der Verschiebung des Messstabs und daraus der Verschiebung des Druckelements relativ zum Abstützelement, insbesondere des Überstandes des Druckelementes bzw. seiner Kontaktfläche über das Abstützelement, aufweisen.

Die Vorrichtung kann derart ausgestaltet sein, dass die Druckerzeugungsvorrichtung elektronisch oder mechanisch zur Ausübung der Druckkraft betätigbar ist.

Ferner kann die Vorrichtung dahingehend ausgebildet sein, dass die Druckkraft einstellbar, insbesondere mechanisch oder elektronisch einstellbar, ist.

Die vorliegende Erfindung schließt auch ein Verfahren zur Messung einer Kompressibilität eines biologischen Gewebes mit einer erfindungsgemäßen Vorrichtung ein. Bei diesem Verfahren wird das Abstützelement zunächst auf ein biologisches Gewebe aufgebracht. Die Druckerzeugungsvorrichtung übt dann Druck auf das Druckelement aus, sodass das Druckelement relativ zum Abstützelement verschoben wird. Mittels der Messeinheit kann dann die Verschiebung des Druckelements relativ zum Abstützelement, insbesondere der Überstand des Druckelements über das Abstützelement während der Ausübung des Drucks auf das Druckelement, gemessen werden.

Im Folgenden wird ein konkretes Beispiel einer erfindungsgemäßen Vorrichtung genauer beschrieben. Dabei sind die in dem Beispiel beschriebenen Merkmale der erfindungsgemäßen Vorrichtung und des erfindungsgemäßen Verfahrens nicht allein auf das konkrete Beispiel beschränkt, sondern können auch unabhängig davon realisiert sein.

Es zeigen
- Figur 1: eine erfindungsgemäße Vorrichtung in einer Perspektivansicht,
- Figur 2: eine erfindungsgemäße Vorrichtung in einer Vorderansicht.

In der nachfolgenden Beschreibung der Figuren bezeichnen gleiche oder ähnliche Bezugszeichen gleiche oder ähnliche Elemente. Folgende Bezugszeichen wurden verwendet:

| | |
|---|---|
| Vorrichtung | 1 |
| Abstützelement | 2 |
| Druckelement | 3 |
| Messeinheit | 4 |
| Knopf | 5 |
| Grundplattenbolzen | 6 |
| Bügel | 7 |
| Grundplatte | 8 |
| Gasdruckfeder | 9 |
| Gasdruckfederaufnahme | 10 |
| Messstab | 11 |
| Feststellschraube | 12 |
| Anzeige | 13 |
| Loch | 14 |
| Gasdruckfedergehäuse | 15 |

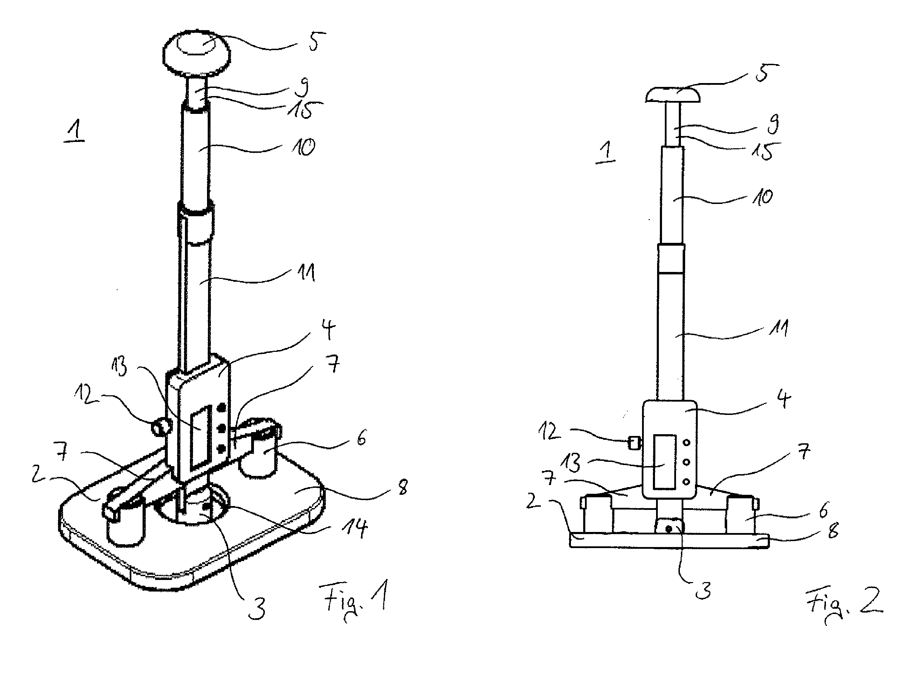

Figur 1 und Figur 2 zeigen eine erfindungsgemäße Vorrichtung 1 zur Messung der Kompressibilität eines biologischen Gewebes, insbesondere eines humanen Gewebes in einer Perspektivansicht (Figur 1) und einer Vorderansicht (Figur 2).

Die Vorrichtung 1 weist eine in etwa rechteckige Grundplatte 8 mit abgerundeten Ecken auf. Die Grundplatte 8 ist mittig mit einem kreisförmigen Loch 14 versehen. Auf der Grundplatte 8 sind zwei zylinderförmige Grundplattenbolzen 6 derart angeordnet, dass die Zylinderachse der Grundplattenbolzen 6 senkrecht zur Grundplatte 8 verlaufen. Die Grundplattenbolzen 6 befinden sich jeweils an gegenüberliegenden Seiten des Lochs 14 und in etwa mittig bezüglich der kurzen Seiten der rechteckförmigen Grundplatte 8. An den der Grundplatte 8 abgewandten Oberseiten der Grundplattenbolzen 6 ist jeweils ein Arm 7 befestigt, der sich in Richtung des Lochs 14 und oberhalb des Lochs 14 erstreckt. Die Arme 7 sind über eine Messeinheit 4 fest miteinander verbunden.

Die Messeinheit 4 ist in etwa quaderförmig ausgebildet, wobei ihre Längsrichtung senkrecht zur Grundplatte verläuft. An ihrer der Grundplatte 8 zugewandten Unterseite ist die Messeinheit 4 mit beiden Armen 7 fest verbunden, sodass sich die Messeinheit 4 zentral über dem Loch 14 befindet. Die Messeinheit 4 weist weiter eine quaderförmige Ausbuchtung im Inneren auf, wobei die quaderförmige Ausbuchtung parallel zur quaderförmigen Messeinheit ausgerichtet ist. Desweiteren weist die Messeinheit 4 an einer einem der Grundplattenbolzen 6 zugewandten Seite eine Feststellschraube 12 auf. Außerdem weist die Messeinheit 4 an einer einer der langen Seiten der rechteckigen Grundplatte 8 zugewandten Vorderseite eine Anzeige 13 auf.

In der quaderförmigen Ausbuchtung der Messeinheit 4 ist ein länglicher quaderförmiger Messstab 11 in Richtung senkrecht zur Grundplatte 8 beweglich gelagert. Der Messstab 11 steht dabei auf der der Grundplatte 8 abgewandten Oberseite der Messeinheit 4 und auf der der Grundplatte 8 zugewandten Unterseite der Messeinheit 4 aus der Messeinheit 4 heraus. An dem der Grundplatte 8 zugewandten Ende des Messstabs 11 ist ein zylinderförmiges Druckelement 3 mittels einer Schraube derart befestigt, dass die Zylinderachse des Druckelements 3 parallel zur Längsausdehnung des Messstabs 11 ausgerichtet ist. Das Druckelement 3 ist mittig bezüglich der Fläche des Lochs 14 ausgerichtet, sodass sich durch die bewegliche Lagerung des Messstabs 11 in der Messeinheit 4 das Druckelement 3 durch das Loch 14 hindurch und somit relativ zur Grundplatte 8 verschieben lässt.

An dem der Grundplatte 8 abgewandten oberen Ende des Messstabs 11 ist eine zylinderförmige Gasdruckfederaufnahme 10 befestigt, deren Zylinderachse parallel zur Längsausdehnung des Messstabs 11 ausgerichtet ist. Die Dicke der zylinderförmigen Gasdruckfederaufnahme 10 entspricht in etwa der Breite des Messstabs 11. Innerhalb der zylinderförmigen Gasdruckfederaufnahme 10 ist eine Gasdruckfeder 9 mit einer Kolbenstange (nicht gezeigt) gelagert, wobei die Kraftrichtung der Gasdruckfeder 9 parallel zur Zylinderachse der Gasdruckfederaufnahme 10 sowie parallel zur Längsausdehnung des Messstabs 11 ausgerichtet ist. An ihrem der Grundplatte 8 abgewandten oberen Ende weist die Gasdruckfeder 9 einen kuppelförmigen Knopf 5 zur Betätigung der Vorrichtung 1 auf.

Eine Messung der Kompressibilität eines biologischen Gewebes, insbesondere eines humanen Gewebes, kann mit der Vorrichtung 1 wie folgt durchgeführt werden. Die Vorrichtung 1 wird mit ihrer Grundplatte 8 an ein Gewebe, beispielsweise an die Haut eines Körperteils, angelegt. Dabei kann die Vorrichtung 1 an der Grundplatte 8, den Grundplattenbolzen 6, den Armen 7 oder an der Messeinheit 4 gehalten werden. Dann wird der Knopf 5 in Richtung der Grundplatte 8 gedrückt. Hierbei federt die Gasdruckfeder 9 ein, d.h. die sich in der Gasdruckfeder 9 befindliche Kolbenstange (hier nicht sichtbar) bewegt sich in das Gehäuse 15 der Gasdruckfeder 9 hinein. Die Kolbenstange der Gasdruckfeder 9 läuft innerhalb der zylinderförmigen Gasdruckfederaufnahme 10 und ist in Druckkontakt mit dem Messstab 11. Durch den Druck der Gasdruckfeder 9 auf die Kolbenstange der Gasdruckfeder 9 übt die Kolbenstange einen Druck auf den Messstab 11 aus. Aufgrund dieses Druckes verschiebt sich der Messstab 11 und mit diesem auch das Druckelement 3 relativ zur Grundplatte 8 und drückt dadurch das Gewebe ein.

Die Gasdruckfeder 9 ist so eingestellt, dass die Druckkraft entlang des gesamten Federwegs konstant ist. Dies gewährleistet, dass jede Messung mit der gleichen Druckkraft vorgenommen werden kann und die Eindrücktiefe des Gewebes lediglich von der Kompressibilität des Gewebes abhängt.

Die Eindrücktiefe des Gewebes kann dann anhand der relativen Verschiebung zwischen dem Messstab 11 und der Messeinheit 4 ermittelt werden. Hierzu ist der Messstab 11 entweder mit einer Skala versehen, die an einem Referenzpunkt der Anzeige 13 ablesbar ist, oder die Messeinheit 4 verfügt über eine digitale Anzeige, auf der die Verschiebung abgelesen werden kann. Aus der der Eindrücktiefe des Gewebes bei definierter Druckkraft kann dann die Kompressibilität des Gewebes bestimmt werden.

Es ist vorteilhaft, wenn während der Messung bzw. der Betätigung des Knopfes 5 und somit der Druckausübung die Feststellschraube 12 betätigt und dadurch die relative Verschiebung zwischen der Messeinheit 4 und dem Messstab 11 fixiert wird. Auf diese Weise kann auch nach Beendigung der Messung und Abstellen der Vorrichtung 1 die relative Verschiebung noch abgelesen werden.

## Patentansprüche

1. Vorrichtung zur Messung der Kompressibilität eines biologischen Gewebes, insbesondere humanen Gewebes, mit einem Abstützelement zur Abstützung der Vorrichtung auf dem Gewebe,
einem gegenüber dem Abstützelement verschiebbar gelagerten Druckelement zur Ausübung eines Drucks auf das Gewebe, und einer Messeinheit zur Bestimmung der Verschiebung des Druckelements relativ zum Abstützelement und hieraus der Kompressibilität des biologischen Gewebes,
**gekennzeichnet durch**
eine Druckerzeugungsvorrichtung zur Ausübung einer von der Verschiebung des Druckelementes im Wesentlichen unabhängigen einheitlichen Druckkraft auf das Druckelement in Richtung des Gewebes.

2. Vorrichtung nach dem vorhergehenden Anspruch, **dadurch gekennzeichnet, dass** die Druckerzeugungsvorrichtung eine Feder, insbesondere eine Kolbenschubeinheit, insbesondere eine Gasdruckfeder, einen Pneumatikzylinder, eine Schraubenfeder, eine Öldruckfeder, eine Elastomerfeder und/oder einen Gummizug aufweist.

3. Vorrichtung nach einem der vorhergehenden Ansprüche, **dadurch gekennzeichnet, dass** das Abstützelement eine flächige Grundplatte zur Auflage auf das biologische Gewebe aufweist.

4. Vorrichtung nach dem vorhergehenden Anspruch, **dadurch gekennzeichnet, dass** die Grundplatte eine ebene Oberfläche, eine anatomisch geformte Oberfläche oder eine gewölbte Oberfläche und/oder eine Oberfläche mit einer ringförmigen Gitterstruktur zur Auflage auf das biologische Gewebe aufweist.

5. Vorrichtung nach einem der vorhergehenden Ansprüche, **dadurch gekennzeichnet, dass** das Druckelement eine Kontaktfläche zur Ausübung des Drucks auf das Gewebe mit einer Fläche zwischen 0,05 cm² und 10 cm², vorteilhafterweise zwischen 0,05 cm² und 5 cm², besonders bevorzugt zwischen 0,05 cm² und 2 cm², vorteilhafterweise von 0,1 cm² oder 0,5 cm² oder 1 cm² oder 1,5 cm² oder 2 cm² aufweist.

6. Vorrichtung nach dem vorhergehenden Anspruch, **dadurch gekennzeichnet, dass** die Kontaktfläche anatomisch geformt, eben, gewölbt, kreisförmig und/oder oval ist.

7. Vorrichtung nach einem der vorhergehenden Ansprüche, **dadurch gekennzeichnet, dass** die Druckerzeugungsvorrichtung in einem Hohlzylinder gelagert ist, sodass die Kraftrichtung der Druckerzeugungsvorrichtung und die Symmetrieachse des Hohlzylinders parallel zueinander liegen.

8. Vorrichtung nach dem vorhergehenden Anspruch, **dadurch gekennzeichnet, dass** innerhalb des Hohlzylinders eine in Richtung der Symmetrieachse bewegliche Kolbenstange angeordnet ist, die Teil der Druckerzeugungsvorrichtung ist und mit dem Druckelement derart in Kontakt steht, daß sie die Druckkraft der Druckerzeugungsvorrichtung auf das Druckelement überträgt.

9. Vorrichtung nach einem der beiden vorhergehenden Ansprüche, **dadurch gekennzeichnet, dass** die Messeinheit einen Messstab aufweist, der zwischen dem Druckelement und dem Hohlzylinder angeordnet ist und mit dem Druckelement und der Druckerzeugungsvorrichtung derart in Kontakt steht, dass er die Druckkraft von der Druckerzeugungsvorrichtung auf das Druckelement überträgt.

10. Vorrichtung nach dem vorhergehenden Anspruch, **dadurch gekennzeichnet, dass** die Messeinheit eine Messvorrichtung zur Messung der Verschiebung des Messstabs und daraus der Verschiebung des Druckelements relativ zum Abstützelement, insbesondere des Überstandes des Druckelementes bzw. seiner Kontaktfläche über das Abstützelement, aufweist.

11. Vorrichtung nach einem der beiden vorhergehenden Ansprüche, **dadurch gekennzeichnet, dass** an dem Messstab eine Feststellschraube zur Fixierung der Position des Messstabs in Richtung der Kraftrichtung der Druckkraft angeordnet ist.

12. Vorrichtung nach einem der vorhergehenden Ansprüche, **dadurch gekennzeichnet, dass** die Druckerzeugungsvorrichtung elektronisch oder mechanisch zur Ausübung der Druckkraft betätigbar ist.

13. Vorrichtung nach einem der vorhergehenden Ansprüche, **dadurch gekennzeichnet, dass** die Druckkraft einstellbar, insbesondere mechanisch oder elektronisch einstellbar, ist.

14. Verfahren zur Messung einer Kompressibilität eines biologischen Gewebes mit einer Vorrichtung nach einem der vorhergehenden Ansprüche, wobei das Abstützelement auf ein biologisches Gewebe aufgebracht wird, die Druckerzeugungsvorrichtung die Druckkraft auf das Druckelement ausübt, so dass das Druckelement relativ zum Abstützelement verschoben wird, und mittels der Messeinheit die Verschiebung des Druckelements relativ zum Abstützelement, insbesondere der Überstand des Druckelements über das Abstützelement während der Ausübung des Drucks auf das Druckelement, gemessen wird.
